# EUROPÄISCHE PATENTSCHRIFT

(11) **EP 1 726 274 B1**
(45) Veröffentlichungstag und Bekanntmachung des Hinweises auf die Patenterteilung: **26.03.2008**
(21) Anmeldenummer: 06017467.9
(22) Anmeldetag: 09.03.2001
(51) Int. Cl.: A61F 2/46

(54) **Kanüle für eine Vorrichtung zum Applizieren von Knochenzement**
Cannula for a device for applying bone cement
Canule pour un dispositif pour appliquer du ciment osseux

(30) Priorität: 25.05.2000 DE 10025898; 22.12.2000 DE 10064202
(43) Veröffentlichungstag der Anmeldung: 29.11.2006
(62) Teilanmeldung aus: 01105912.8
(73) Patentinhaber: Pajunk GmbH & Co. KG Besitzverwaltung, 78187 Geisingen (DE)
(72) Erfinder: Pajunk, Heinrich, 78187 Geisingen (DE); Pajunk, Horst, 78187 Geisingen (DE)
(74) Vertreter: Manitz, Finsterwald & Partner GbR

(56) Entgegenhaltungen:
- EP-A- 1 074 231
- FR-A- 577 367
- US-A- 2 711 733
- US-A- 4 576 152
- US-A- 5 051 482
- US-A- 5 398 483
- US-A- 6 048 346

## Beschreibung

Die vorliegende Erfindung betrifft eine Kanüle für eine Vorrichtung zum Applizieren von Knochenzement gemäß dem Oberbegriff des Anspruchs 1. Eine derartige Kanüle ist aus der US 6,048,346 bekannt.

Weiterhin wird eine Vorrichtung zum Applizieren von Knochenzement mit einem Gehäuse, das einen Zylinder zur Aufnahme des Knochenzements umfaßt, und mit einem in dem Zylinder längsverschiebbar angeordneten Kolben, durch den der Knochenzement durch eine in dem Zylinder ausgebildete Austrittsöffnung herauspreßbar ist, wobei der Kolben zum Applizieren des Knochenzements unter hohem Druck durch eine Schraubbewegung in dem Zylinder längsverschiebbar ist, beschrieben.

Applikationsvorrichtungen dieser Art werden verwendet, wenn Knochenstrukturen beispielsweise durch Knochenkrebs oder durch Osteoporose zersetzt oder brüchig geworden sind. Mit entsprechenden Vorrichtungen ist die Applikation von Knochenzement unmittelbar in die betroffenen Knochenstrukturen möglich, wodurch die verfestigt werden.

Bei der Applikation sind dabei mehrere Anforderungen zu beachten. Zum einen muß die Befüllung der Applikationsvorrichtung sowie die Applikation in die betroffenen Knochenstrukturen sehr schnell, innerhalb weniger Minuten erfolgen, da die üblicherweise verwendeten Knochenzemente 6 bis 7 Minuten nach dem Anrühren auszuhärten beginnen. Zum anderen muß der Knochenzement mit sehr hohem Druck appliziert werden, da andernfalls eine ausreichende Durchdringung der Knochenstrukturen nicht gewährleistet ist. Letztlich muß die Applikation des Knochenzements gut steuerbar sein, da insbesondere bei der Applikation im Bereich der Wirbelsäule ein Fehlleiten des Knochenzements zu irreversiblen Schädigungen beispielsweise von Nerven führen kann.

Es ist eine Aufgabe der vorliegenden Erfindung, eine Kanüle für eine Applikationsvorrichtung anzugeben, mit der die Applikation in kurzer Zeit durchgeführt werden kann, wobei gleichzeitig der erforderliche hohe Druck aufgebaut werden kann und eine Steuerbarkeit des applizierten Knochenzements möglich ist.

Diese Aufgabe wird durch die Merkmale des Anspruchs 1 gelöst.

Im Rahmen dieser Anmeldung wird dabei der Begriff "proximal" mit der Bedeutung "zum Körper des Arztes hin gelegen" verwendet. Dementsprechend wird der Begriff "distal" mit der Bedeutung "vom Körper des Arztes entfernt gelegen" verwendet.

Die Vorrichtung ist zwischen der Verschiebung des Kolbens durch die Schraubbewegung und einer direkten Verschiebung in Längsrichtung ohne Schraubbewegung umstellbar. Durch die Umstellbarkeit der Applikationsvorrichtung ist es möglich, daß beispielsweise das Auffüllen des Zylinders durch eine Aufziehbewegung des Kolbens, d.h. eine direkte Verschiebung des Kolbens in Längsrichtung, in sehr kurzer Zeit erfolgt. In umgekehrter Weise kann anschließend der im Zylinder vorhandene, flüssige Knochenzement durch direktes Vorschieben des Kolbens in kurzer Zeit so lange appliziert werden, bis der entstehende Gegendruck so groß wird, daß er durch die direkte Vorschubbewegung nicht mehr überwunden werden kann. In diesem Moment wird die Applikationsvorrichtung auf einen Modus "Verschiebung des Kolbens durch Schraubbewegung" umgestellt, da durch die Schraubbewegung ein wesentlich höherer Druck auf den Kolben und damit auf den zu applizierenden Knochenzement ausgeübt werden kann als mit einer direkten Vorschubbewegung.

Die Vorschubgeschwindigkeit ist bei der Verschiebung durch die Schraubbewegung zwar deutlich geringer als bei der direkten Verschiebung in Längsrichtung; da bis zum Erreichen des beschriebenen hohen Drucks jedoch sowohl das Auffüllen des Zylinders als auch das Applizieren des flüssigen Knochenzements durch die direkte Längsverschiebung des Kolbens in sehr kurzer Zeit erfolgen kann, ist üblicherweise zu dem Zeitpunkt, an dem auf die weitere Applizierung durch Schraubbewegung umgestellt werden muß, noch ausreichend Zeit vorhanden, um die Applikation zu beenden, bevor der Knochenzement auszuhärten beginnt.

Ein weiterer Vorteil der Applikationsvorrichtung besteht darin, daß der während der Applikation mit Schraubbewegung aufgebaute hohe Druck sehr schnell, d.h. innerhalb von Sekundenbruchteilen abgebaut werden kann. Dies ist beispielsweise dann erforderlich, wenn bei der Beobachtung des aus der Kanüle austretenden Knochenzements beispielsweise an einen Fluoroskop, eine Fehlleitung des Knochenzements erkannt wird. In diesem Fall kann durch einfaches Umstellen der Vorrichtung auf die direkte Längsverschiebbarkeit des Kolbens erreicht werden, daß durch den hohe Druck der Kolben zurückgeschoben und damit automatisch der hohe Druck abgebaut wird. Auf diese Weise wird der fehlgeleitete Austritt des Knochenzements aus dem distalen Ende der Kanüle unmittelbar gestoppt.

Nach einer vorteilhaften Ausführungsform umfaßt der Kolben einen Eingriffsabschnitt mit einem Schraubgewinde, das in eine am Gehäuse vorgesehene Gegenverzahnung eingreift, so daß beim Verdrehen des Eingriffsäbschnitts die Längsverschiebung des Kolbens erfolgt. Die Gegenverzahnung kann dabei insbesondere als Zahnstange ausgebildet sein. Auf diese Weise wird ein sehr einfacher, kostengünstiger und funktionssicherer Aufbau einer Applikationsvorrichtung erreicht. Insbesondere wird in dem Betriebsmodus "Verschiebung durch Schraubbewegung" durch die ineinandergreifenden Verzahnungen automatisch eine direkte Verschiebung in Längsrichtung verhindert, so daß die mit jeder Umdrehung erzielter Erhöhung des angewendeten Drucks automatisch gesichert ist.

Nach einer weiteren bevorzugten Ausführungsform sind das Schraubgewinde und die Gegenverzahnung entkoppelbar. Dabei kann vorteilhaft zur Entkopplung die Gegenverzahung in einer Richtung im wesentlichen senkrecht zur Verschieberichtung des Kolbens zwischen einer Verriegelungsstellung und einer Freigabestellung bewegbar sein. Durch diese Ausbildung ist eine einfache und schnelle Umschaltung von dem Betriebszustand "Verschieben durch Schraubbewegung" in den Betriebszustand "direkte Verschiebung in Längsrichtung" und zurück möglich, indem beispielsweise über eine am Gehäuse angebrachte Betätigungseinheit die Gegenverzahnung in die Freigabestellung verschoben wird.

Vorteilhaft wird die Gegenverzahnung unter Vorspannung gegen das Schraubgewinde gedrückt. Dadurch ist gewährleistet, daß der aufgebaute Druck solange automatisch gesichert ist, bis die Gegenverzahnung entgegen der Vorspannung bewegt wird. Diese Vorspannung kann beispielsweise durch eine Federbeaufschlagung erfolgen.

Nach einer weiteren vorteilhaften Ausführungsform umgreift die Gegenverzahnung das Schraubgewinde bereichsweise, insbesondere hinterschneidungsfrei. Durch eine bereichsweise Umgreifung des Schraubgewindes wird eine vergrößerte Kontaktfläche zwischen den unter hohem Druck aneinander anliegenden Zähnen der Gegenverzahnung und dem Gewindeprofil des Schraubgewindes erreicht, so daß die Stabilität der Vorrichtung erhöht ist. Durch das hinterschneidungsfreie Umgreifen ist dabei gewährleistet, daß weiterhin eine einfache Entkopplung von Schraubgewinde und Gegenverzahnung, beispielsweise durch einfaches seitliches Verschieben der Gegenverzahnung möglich ist.

Bevorzugt ist im entkoppelten Zustand der Kolben im wesentlichen frei in dem Zylinder längsverschiebbar. Die freie Verschiebbarkeit des Kolbens ist dabei im wesentlichen nur durch eine Dichtung beeinträchtigt, die üblicherweise zwischen dem Kolbenumfang und der Innenwand des Zylinders zur Abdichtung vorgesehen ist.

Nach einer weiteren vorteilhaften Ausführungsform schließen die beim Applizieren des Knochenzements unter Druck aneinander anliegenden Zahnflanken der Gegenverzahnung und/oder die Flanken des Gewindeprofils des Schraubgewindes einen Winkel von kleiner oder gleich ca. 90° mit der zur Verschieberichtung parallel verlaufenden Längsachse des Eingriffsabschnitts ein. Durch diese spezielle Ausbildung der Flanken ist gewährleistet, daß auch bei Anwendung sehr hoher Drücke kein Überschnappen einzelner Zähne auftritt, wie es beispielsweise bei üblichen, abgeschrägten Flanken der Fall sein kann, bei denen der Winkel zwischen den Flanken und der Längsachse des Eingriffsabschnitts größer als 90° ist. Beträgt der Winkel im wesentlichen gleich 90°, so kann die Gegenverzahnung zum Entkoppeln von dem Schraubgewinde durch eine Schiebebewegung senkrecht zur Bewegungsrichtung des Eingriffsabschnitts verschoben werden. Sind die Winkel kleiner als 90°, so ist eine Entkopplung durch eine entsprechende Verschiebung der Gegenverzahnung schräg zur Längsachse des Eingriffsabschnitts möglich.

Bevorzugt sind der Kolben und der Eingriffsabschnitt einstückig ausgebildet. Bei einer einstückigen Ausbildung muß gewährleistet sein, daß der Kolben in dem Zylinder verdrehbar ist, um auf diese Weise die Verschraubung des Eingriffsabschnitts zu ermöglichen. Bei dieser Ausführungsform wird somit die Längsverschiebung des Kolbens unmittelbar durch Einschrauben des Kolbens erzielt.

Es ist auch möglich, daß der Kolben zweiteilig ausgebildet ist, so daß der Eingriffsabschnitt ein separates Teil bildet. In diesem Fall sind beide Teile miteinander insbesondere gegeneinander verdrehbar verbunden. Bei dieser Ausführung ist es möglich, daß bei der Schraubbewegung lediglich der Eingriffsabschnitt verdreht wird, während der Kolben unverdreht durch den sich vorwärtschraubenden Eingriffsabschnitt innerhalb des Zylinders nach vorne verschoben wird. Während in diesem Fall die Querschnittsflächen des Kolbens und des Zylinders zwar komplementär zueinander, in der Form jedoch grundsätzlich beliebig sein können, beispielsweise oval oder eine mehrkantige Form besitzen können, ist bei der einstückigen Ausbildung von Kolben und Eingriffsabschnitt der Zylinder üblicherweise als Kreiszylinder ausgebildet, um auf diese Weise eine Verdrehung des Kolbens im Zylinder zusammen mit dem Eingriffsabschnitt zu ermöglichen.

Nach einer weiteren vorteilhaften Ausführungsform ist an der Austrittsöffnung des Zylinders eine Kanüle befestigbar. Diese Kanüle ist bevorzugt lösbar befestigt, da auf diese Weise in einem ersten Verfahrensschritt die Kanüle ohne Applikationsvorrichtung in den Patienten eingebracht und positioniert werden kann, während erst nach erfolgreicher Plazierung in einem zweiten Verfahrensschritt die Applikationsvorrichtung beispielsweise über eine ebenfalls an der Austrittsöffnung befestigbaren Aufziehkanüle mit dem flüssigen Knochenzement befüllt wird. Nach Entfernen der Aufziehkanüle kann anschließend die Applikationsvorrichtung an der bereits eingesetzten Injektionskanüle befestigt werden und der Knochenzement in der zuvor beschriebenen Weise appliziert werden.

Die erfindungsgemäße Kanüle hat den Vorteil, daß aufgrund ihrer asymmetrischen Spitze sowie der seitlich nach außen ragenden Angriffselemente bereits beim Einbringen der Kanüle eine exakte Positionierung vorgenommen werden kann. Übliche Kanülen weisen eine symmetrische Spitze auf und können beim Einbringen bezüglich der Einbringrichtung nicht verändert werden. Bei der erfindungsgemäßen Kanüle kann hingegen die asymmetrische Spitze durch ein Verdrehen der teilweise eingeführten Kanüle über die seitlich nach außen ragenden Angriffselemente so positioniert werden, daß eine Bewegung der Kanüle beim weiteren Einbringen in die gewünschte Richtung erreicht wird. Durch die vorauseilende asymmetrische Spitze wird die Kanüle beim Einbringen immer geringfügig in die Richtung seitlich abweichen, zu der die Spitze gerade hin gelegen ist. Durch wiederholtes abwechselndes Einbringen und Verdrehen ist somit mit der erfindungsgemäß ausgebildeten Kanüle eine verbesserte Positionierung sowie ein nachträgliches Korrigieren der Position noch während des Einbringens möglich.

Weiterhin ist es mit der erfindungsgemäßen Kanüle möglich, nach Aushärtung des applizierten Knochenzements durch Verdrehen der noch im Körper befindlichen Kanüle über die Angriffselemente um ihre Längsachse ein Abdrehen bzw. Abscheren des sich noch innerhalb der Kanüle befindenden aushärtenden Knochenzements von dem in die Knochenstruktur applizierten Knochenzements zu ermöglichen. Dadurch ist sichergestellt, daß der sich innerhalb der Kanüle befindende Knochenzement beim Herausziehen der Kanüle in dieser verbleibt und sicher mit dieser zusammen aus dem Gewebe entnommen wird.

Bevorzugt ist die die Umrandung der Öffnung bildende Kante des Kanülenendes als Schneide geschliffen. Dadurch ist auch bei bereits vollständig ausgehärtetem Knochenzement eine sicher Abscherung des innerhalb der Kanüle angeordneten Materials gewährleistet.

Nach einer weiteren vorteilhaften Ausführungsform der Erfindung erstreckt sich die Durchtrittsfläche der Öffnung schräg zur Längsachse der Kanüle. Auf diese Weise kann die Bewegungsrichtung des aus der Öffnung in der Kanülenspitze austretenden Knochenzements gesteuert werden. Durch Verdrehen der Kanüle über die Angriffselemente kann die Öffnung so plaziert werden, daß das austretende Material in die gewünschte Richtung fließt. Bei Kanülen mit einer zentrischen Öffnung, wie sie aus dem Stand der Technik bekannt sind, tritt der Knochenzement hingegen immer in Längsrichtung nach vorne aus, so daß durch ein Verdrehen der Kanüle die Austrittsrichtung nicht geändert werden kann.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnungen näher beschrieben; in diesen zeigen:
- Fig. 1: einen teilweise aufgerissenen Querschnitt einer Applikationsvorrichtung,
- Fig. 2: eine Detailansicht einer Vorrichtung nach Fig. 1,
- Fig. 3: einen teilweise aufgerissenen Querschnitt der Vorrichtung nach Fig. 1,
- Fig. 4: eine erfindungsgemäß ausgebildete Kanüle,
- Fig. 5: eine Detailansicht der Kanüle nach Fig. 4,
- Fig. 6: eine weitere Ausführungsform einer erfindungsgemäß ausgebildeten Kanüle und
- Fig. 7: eine Detailansicht der Kanüle nach Fig. 6.

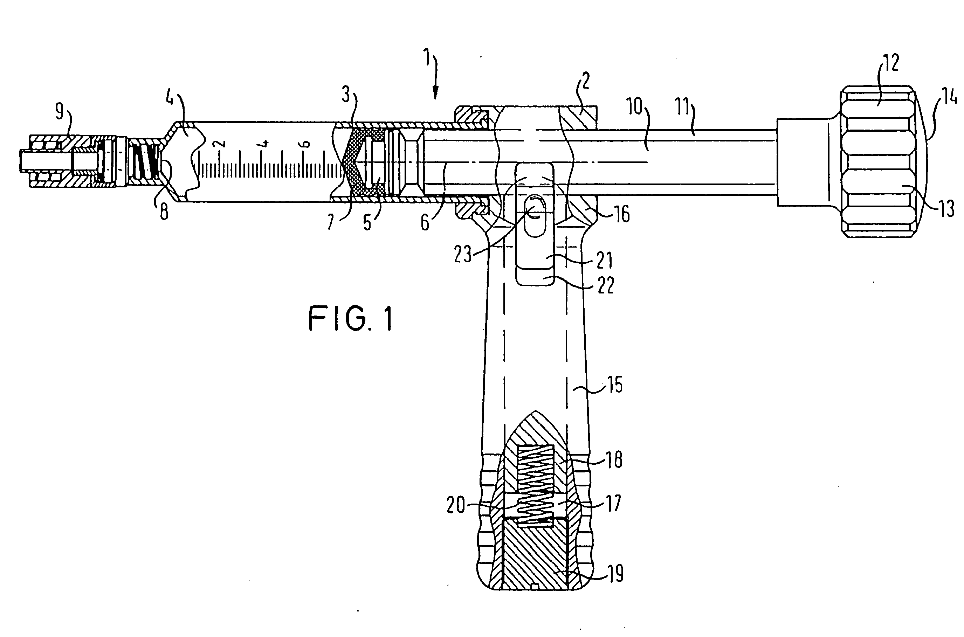

Fig. 1 zeigt eine pistolenförmige Applikationsvorrichtung 1 mit einem Gehäuse 2, dessen mittlerer Bereich als Zylinder 3 zur Aufnahme von Knochenzement in seinem Inneren 4 ausgebildet ist. Innerhalb des Zylinders 3 ist ein Kolben 5 in Richtung seiner Längsachse 6 verschiebbar gelagert, wobei der Kolben 5 mit einem Abdichtungsmittel 7 gegenüber der Innenwand des Zylinders 3 abgedichtet ist. Am distalen Ende des Zylinders 3 ist eine Austrittsöffnung 8 ausgebildet, an der ein beispielsweise als große Luer Lock-Verbindung ausgebildetes Kopplungselement 9 zum Ankoppeln einer Kanüle vorgesehen ist.

Der Kolben 5 ist einstückig mit einem langgestreckten Schaft 10 ausgebildet, der zum Bilden eines Eingriffsabschnitt an seiner Außenseite mit einem Schraubgewinde 11 versehen ist. Am proximalen Ende des Schaftes 10 ist ein als Dreh-/Schiebeknopf ausgebildetes Betätigungselement 12 vorgesehen, das an seiner Umfangsfläche Vertiefungen 13 und an seinem stirnseitigen Ende eine gewölbte Angriffsfläche 14 besitzt.

Das Gehäuse 2 umfaßt weiterhin einen sich in Fig. 1 nach unten erstreckenden, einen Handgriff 15 bildenden Abschnitt, durch dessen oberen Bereich 16 der Schaft 10 des Kolbens 5 hindurchgeführt ist und der einen Hohlraum 17 umfaßt, in dem ein Verriegelungselement 18 längsverschiebbar angeordnet ist.

Der Hohlraum 17 ist zum freien Ende des Handgriffs 15 hin offen ausgebildet und dort mit einem beispielsweise eingeschraubten Verschlußelement 19 verschlossen. Das Verschlußelement 19 dient gleichzeitig als Abstützelement für eine Schraubenfeder 20, mit der das Verriegelungselement 18 in Richtung des oberen Bereichs 16 des Handgriffs 15 gedrängt wird, so daß es an dem Schaft 10 des Kolbens 5 zur Anlage kommt.

Mit dem Verriegelungselement 18 ist ein als Schieber ausgebildetes Entriegelungselement 21 verbunden, das in einer Führungsausnehmung 22 an der Außenseite des Gehäuses 2 verschiebbar geführt ist. Das Entriegelungselement ist über einen Bolzen 23 mit dem Verriegelungselement 18 so verbunden, daß bei einer Verschiebung des Entriegelungselements 21 in Richtung des freien Endes des Handgriffs 15 das Verriegelungselement 18 entgegen der Federkraft der Feder 20 verschoben wird.

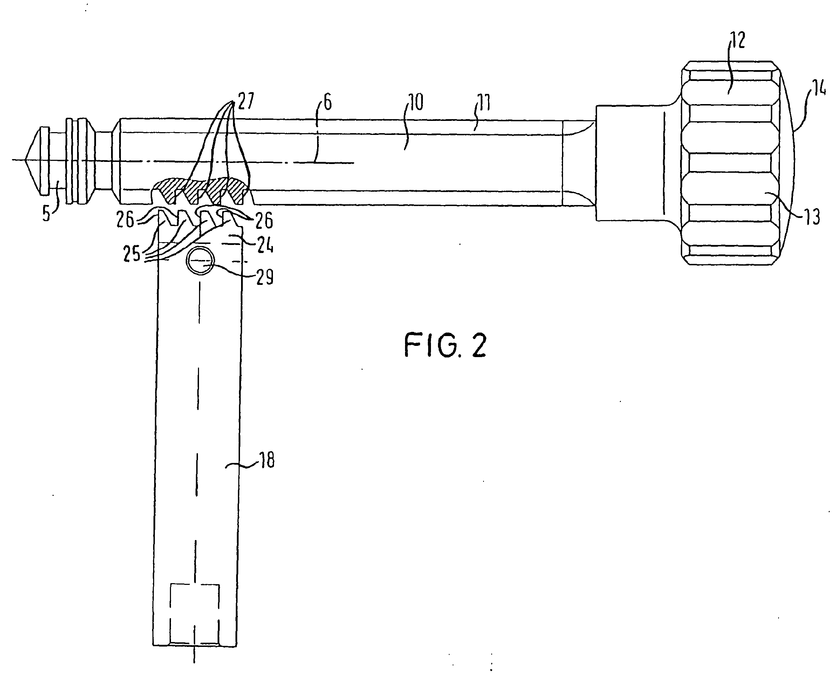

Das Zusammenwirken des Verriegelungselements 18 mit dem Schaft 10 des Kolbens 5 ist in Fig. 2 deutlicher dargestellt.

In Fig. 2 ist zu erkennen, daß das zum Schaft 10 hin gelegene Ende des Verriegelungselements 18 als Zahnstange 24 ausgebildet ist, dessen Zähne 25 eine Gegenverzahnung für das Schraubgewinde 11 des Schaftes 10 bilden.

Wird das Entriegelungselement 21 zum freien Ende des Handgriffs 15 nach unten verschoben, so wird über den Bolzen 23 das Verriegelungselement 18 entgegen der Kraft der Feder 20 nach unten verschoben, bis das Verriegelungselement 18 und der Schaft 10 entkoppelt sind, wie es in Fig. 2 dargestellt ist. In diesem Zustand kann der Schaft 10 und damit der Kolben 5 in dem Zylinder 3 direkt in Längsrichtung im wesentlichen frei verschoben werden, indem beispielsweise mit der Handfläche auf die Angriffsfläche 14 gedrückt wird. Lediglich durch die vorhandene Reibung zwischen dem Abdichtungsmittel 7 und der Innenwand des Zylinders 3 wird diese Verschiebbarkeit etwas beeinträchtigt.

Wird das Entriegelungselement 21 wieder freigegeben, so wird das Verriegelungselement 18 aufgrund der Kraft der Feder 20 in Richtung des Schafts 10 gedrängt, bis die Zähne 25 der Zahnstange 24 mit dem Schraubgewinde 11 des Schaftes 10 in Eingriff sind. In diesem Zustand ist eine Längsverschiebung des Schaftes 10 und damit des Kolbens 5 lediglich durch eine Verschraubung des Schafts 10 möglich, wobei dies bevorzugt über das als Schraubknopf ausgebildete Betätigungselement 12 erfolgt. In diesem Zustand erfolgt durch die Schraubbewegung zwar nur jeweils ein relativ geringer Vorschub des Kolbens 5, der durch die Schraubbewegung aufbringbare Druck auf den im Inneren 4 des Zylinders 3 angeordneten Knochenzement ist jedoch wesentlich höher als er durch eine direkte Verschiebung in Längsrichtung des Schaftes 10 bei von dem Verriegelungselement 18 entkoppelten Schaft 10 erzeugt werden kann.

Wie Fig. 2 zu entnehmen ist, sind die bei einem Hineinschrauben des Kolbens 5 in den Zylinder 3 aneinander anliegenden Flanken 26, 27 des Schraubgewindes 11 bzw. der Zähne 25 im wesentlichen senkrecht zu der Längsachse 6 verlaufend ausgebildet. Dadurch wird erreicht, daß die bei einem Hineinschrauben des Kolbens 5 entstehenden hohen Druckkräfte zwischen den Flanken 26 und 27 vollständig aufgenommen werden, ohne daß eine Kraftkomponente in einer Richtung senkrecht zur Längsachse auf die Flanken 27 wirkt, die ein Verschieben des Verriegelungselements 18 entgegen der Kraft der Feder 20 bewirken könnte. Ein ungewolltes Entkoppeln des Verriegelungselements 18 von dem Schaft 10 ist damit auch bei Auftreten eines sehr hohen Drucks ausgeschlossen.

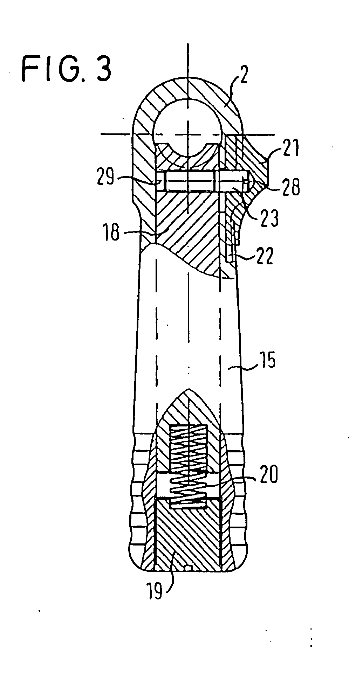

Aus dem Teilquerschnitt gemäß Fig. 3 ist zu erkennen, daß die Zähne 25 der Zahnstange 24 im Querschnitt teilringförmig ausgebildet sind und somit einen vergrößerten Kontaktbereich zu dem Schraubgewinde 11 schaffen. Dadurch ist gewährleistet, daß die zwischen den Zähnen 25 und dem Schraubgewinde 11 auftretende Kraft auf eine möglichst große Fläche verteilt wird, so daß ein Ausbrechen der Zähne 25 oder des Schraubgewindes 11 vermieden wird.

Weiterhin ist in Fig. 3 die Kopplung des Verriegelungselements 18 mit dem Entriegelungselements 21 über den Bolzen 23 erkennbar. Sowohl das Entriegelungselements 21 als auch das Verriegelungselement 18 besitzen dazu jeweils eine Bohrung 28, 29, in die der Bolzen 23 jeweils mit einem Ende eingreift. Auf diese Weise wird eine direkte Kopplung zwischen dem Entriegelungselement 21 und dem Verriegelungselement 18 geschaffen.

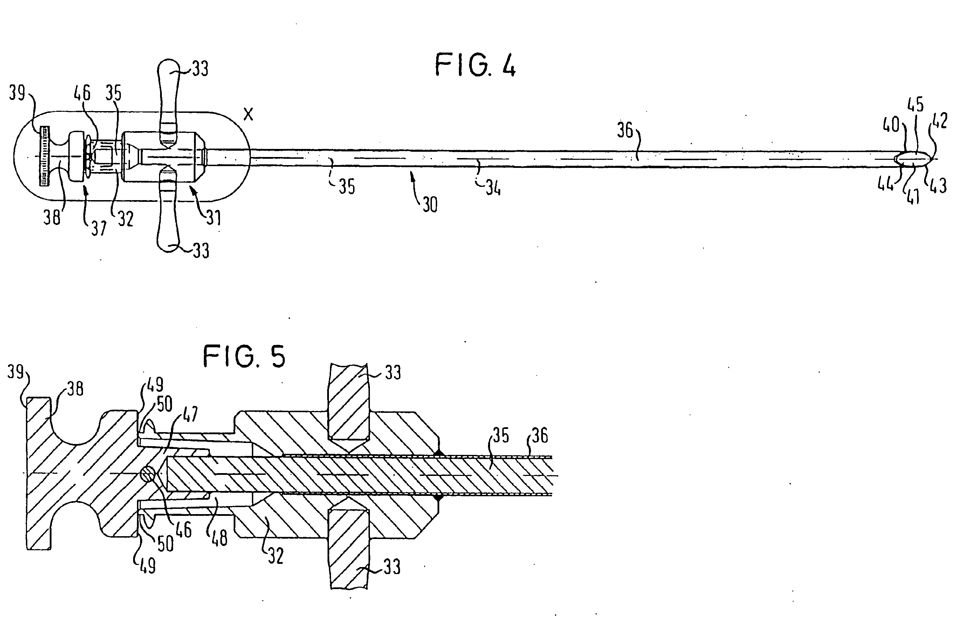

Fig. 4 zeigt eine erfindungsgemäß ausgebildete Kanüle, die beispielsweise mit einer Vorrichtung gemäß Fig. 1 verbunden werden kann. Die Kanüle 30 besitzt dazu an ihrem proximalen Ende 31 ein beispielsweise als große Luer Lock-Verbindung ausgebildetes Kopplungselement 32, das mit dem entsprechenden Kopplungselement 9 (siehe Fig. 1) abdichtend verbunden werden kann. Das Kopplungselement ist beispielsweise aus Metall hergestellt, um die beim Einschlagen der Kanüle auftretenden Kräfte aufnehmen zu können.

An dem proximalen Ende 31 der Kanüle 30 sind zwei sich radial nach außen erstreckende, stiftförmige Angriffselemente 33 vorgesehen, über die die eingesetzte Kanüle 30 in einfacher Weise sowohl um ihre Längsachse 34 verdreht als auch in Richtung der Längsachse 34 aus dem Körper des Patienten wieder herausgezogen werden kann.

Weiterhin ist in Fig. 4 ein Mandrin 35 dargestellt, der in das Rohr 36 der Kanüle 30 eingesetzt ist und an seinem proximalen Ende 37 ein Abschlußelement 38 umfaßt. Das Abschlußeelement 38 ist zum einen zum Halten des Mandrins 35 beim Einsetzen in die Kanüle 30 und beim Herausziehen aus der Kanüle 30 verwendbar. Zum anderen kann das Abschlußeelement 38 dazu verwendet werden, daß die Kanüle 30, falls erforderlich, mit einem Eintriebsmittel, beispielsweise einem Hammer, an die gewünschte Position verbracht wird. Dazu ist das stirnseitige Ende des Abschlußelements 38 als Schlagfläche 39 ausgebildet.

An dem distalen Ende 40 der Kanüle 30 ist eine Öffnung 41 vorgesehen, die beispielsweise durch einen Schrägschnitt des Rohrs 36 erzeugbar ist. Durch diesen Schrägschnitt ist das distale Ende 40 der Kanüle 30 bezüglich ihrer Längsachse 34 asymmetrisch ausgebildet, wobei insbesondere die Spitze 42 der Kanüle 30 seitlich der Längsachse 34, d.h. bei der Darstellung gemäß Fig. 4 in einer Ebene hinter der Längsachse 34 zu liegen kommt.

Die die Umrandung der Öffnung 41 bildende Kante 43 des Rohrs 36 ist so geschliffen, daß diese Kante 43 eine Schneide bildet.

Der Mandrin 35 ist an seinem distalen Ende ebenfalls abgeschrägt ausgebildet und so innerhalb der Kanüle 30 angeordnet, daß die entsprechende Schrägfläche 44 mit der ebenfalls schräg angeordneten Austrittsfläche 45 der Öffnung 41 in Deckung ist. Um diese Deckung zu gewährleisten, ist an dem proximalen Ende 37 des Mandrins 35 eine Justiereinheit in Form eines Stiftes 46 vorgesehen, der in eine entsprechende Ausnehmung an dem Kopplungselement 32 eingreift und damit eine Verdrehsicherung zwischen dem Mandrin 35 und der Kanüle 30 bildet.

Der Stift 46 ist in der Detaildarstellung nach Fig. 5 näher zu erkennen. Ebenso ist aus dieser Darstellung die Anordnung des Mandrins 35 innerhalb des Rohrs 36 der Kanüle 30 erkennbar.

Das Abschlußelement 38 besitzt einen Ansatz 47, der in einen Hohlraum 48 in dem Kopplungselement 32 eingreift. Dabei ist der Durchmesser des Ansatzes 47 geringer als die lichte Weite des Hohlraums 48, so daß auch beim Einschlagen der Kanüle 30 mit einem Hammer trotz der hohen Schlagkräfte kein Verklemmen des Abschlußelements 38 und damit des Mandrins 35 mit dem Kopplungselement 32 erfolgen kann. Dadurch ist gewährleistet, daß der Mandrin 35 nach erfolgter Positionierung der Kanüle 30 problemlos entfernt werden kann.

Zur Übertragung der auf die Schlagfläche 39 des Abschlußelements 38 auftreffenden Schlagkräfte auf die Kanüle 30 stützt sich das Abschlußelement 38 über eine Abstützfläche 49 an dem stirnseitigen Ende 50 des Kopplungselements 32 ab.

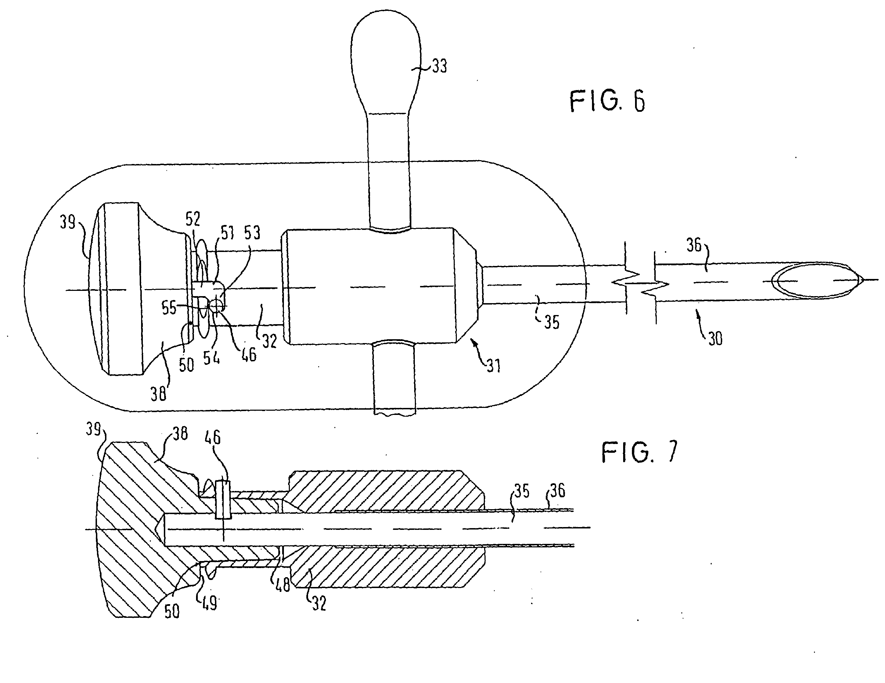

Während die Ausnehmung für den Stift 46 grundsätzlich beispielsweise als in axialer Richtung der Kanüle 30 verlaufender, gerader Schlitz ausgebildet sein kann, ist die Ausnehmung in den Fig. 6 und 7 als abgewinkelter oder L-förmiger Schlitz 51 ausgeführt. Der Schlitz 51 umfaßt einen in axialer Richtung der Kanüle 30 verlaufenden Längsabschnitt 52, der an der ringförmigen, stirnseitigen Fläche 50 des Kopplungselements 32 das offene Ende des Schlitzes 51 bildet, sowie einen in Umfangsrichtung des Kopplungselements 32 verlaufenden Querabschnitt 53, der im wesentlichen senkrecht zum Längsabschnitt 52 angeordnet ist.

Der Schlitz 51 bildet zusammen mit dem Stift 46 einen Bajonettverschluß, wobei im Bereich des freien Endes 54 des Schlitzes 51 eine zur stirnseitigen Fläche 50 des Kopplungselements 32 hin gelegene Vertiefung in Form einer Rastausnehmung 54 vorgesehen ist, in der der Stift 46 bei geschlossenem Bajonettverschluß, je nach Tiefe der Rastausnehmung 55, teilweise oder ganz zu liegen kommt. Auf diese Weise ist trotz des sich in Umfangsrichtung des Kopplungselements 32 erstreckenden Querabschnitts 53 eine Verdrehsicherheit des Mandrins 35 gegenüber der Kanüle 30 gewährleistet.

Die Applikationsvorrichtung 1 sowie die erfindungsgemäß ausgebildete Kanüle werden wir folgt verwendet:

Zunächst wird die Kanüle 30 zusammen mit dem eingesetzten Mandrin 35 in den Körper des Patienten eingeführt, wobei dies im Bedarfsfall unter Zuhilfenahme eines Hammers erfolgt. Bei der Ausführungsform nach den Fig. 6 und 7 wird dabei die Kopplung zwischen dem Mandrin 35 und der Kanüle 30 über den durch den Stift 46 und den Schlitz 51 gebildeten Bajonettverschluß hergestellt. Durch den Bajonettverschluß wird verhindert, daß beim Einschlagen der Kanüle 30 aufgrund einer Federwirkung des Mandrins 35 dieser zurückgedrängt wird und teilweise aus der Kanüle 30 austritt. Ohne entsprechende Sicherung könnte der Mandrin 35 soweit zurückgedrängt werden, daß der Stift 46 aus seiner Längsführung austritt und dadurch die Verdrehsicherheit zwischen Mandrin 35 und Kanüle 30 nicht mehr gegeben ist.

Die jeweilige Position der Kanüle 30 wird beim Einbringen beispielsweise am CT (Computertomograph) verfolgt. Weicht die Position des distalen Endes 40 der Kanüle 30 von der gewünschten Position ab, so wird die Kanüle 30 über die Angriffselemente 33 so verdreht, daß die Spitze 42 in Richtung der gewünschten Position zu liegen kommt. Bei einem weiteren Einbringen der Kanüle 30 wird aufgrund der asymmetrischen Spitze 42 ein gewünschtes Auswandern dieser Spitze 42 in Richtung der gewünschten Position erfolgen.

Ist die Kanüle 30 korrekt positioniert, so wird der Mandrin 35 über das Abschlußelement 38 ergriffen und gegebenenfalls nach Lösen des Bajonettverschlusses aus der Kanüle 30 herausgezogen.

Anschließend wird der verwendete Knochenzement angerührt und über eine mit dem Kopplungselement 9 der Applikationsvorrichtung 1 verbundene Aufziehkanüle in das Innere 4 des Zylinders 3 aufgesogen. Dazu wird das Entriegelungselement entgegen der Kraft der Feder 20 so nach unten verschoben, daß die Zähne 25 außer Eingriff mit dem Schraubgewinde 11 kommen, so daß durch einfaches Zurückziehen des Kolbens 5 mit dem Schaft 11 der Knochenzement über die Aufziehkanüle in den Zylinder 3 gesogen wird.

Anschließend wird die Aufziehkanüle von der Applikationsvorrichtung 1 getrennt und diese mit der bereits in Position gebrachten erfindungsgemäßen Injektionskanüle 30 verbunden.

Im nächsten Verfahrensschritt wird wiederum bei entkoppeltem Verriegelungselement 18 der Kolben 5 mit dem Schaft 10 durch Druckbeaufschlagung der Angriffsfläche 14 des Betätigungselements 12 direkt in den Zylinder 1 hineinverschoben, wodurch das im Inneren 4 des Zylinder 3 angeordnete Zementmaterial über die Kanüle 30 in den Knochen injiziert wird. Durch die Injektion in das Knochenmaterial wird ein ständig wachsender Druck aufgebaut, bis dieser schließlich so hoch wird, daß ein weiteres Applizieren des Knochenzements durch Drücken auf die Angriffsfläche 14 nicht mehr möglich ist.

Zu diesem Zeitpunkt wird das Entriegelungselement 21 freigegeben, so daß das Verriegelungselement 18 durch die Kraft der Feder 20 in Richtung des Schafts 10 verschoben wird, bis die Zähne 25 mit dem Schraubgewinde 11 in Eingriff sind.

Anschließend kann durch Verdrehen des Schafts 10 über das Betätigungselement 12 der Druck im Inneren 4 des Zylinders 3 weiter erhöht werden, so daß der Kolben 5 weiter langsam in das Innere des Zylinders 1 verschoben wird.
Wird beispielsweise durch Beobachtung am Fluoroskop erkannt, daß der applizierte Knochenzement in eine ungewünschte Richtung strömt, kann diese Flußrichtung beispielsweise dadurch geändert werden, daß die Kanüle 30 über die Angriffselemente 33 so verdreht wird, daß die Öffnung 41 in die gewünschte Richtung zeigt.

Ist die aus der Öffnung 41 austretende Menge des Knochenzements aufgrund des hohen Drucks zu groß, so kann dieser Druck unverzüglich durch Verschieben des Entriegelungselements 21 und die dadurch erfolgende Entkopplung und Freigabe des Schafts 10 und des damit verbundenen Kolbens 5 abgebaut werden. Auf diese Weise wird verhindert, daß der Knochenzement an gefährliche Stellen innerhalb des Körpers appliziert wird.

Anschließend kann beispielsweise nach einer Neuorientierung der Kanüle 30 durch Verdrehen über die Angriffselemente 33 der Druck zunächst durch direktes Verschieben und anschließend, wie beschrieben, durch eine weiterführende Schraubbewegung wieder neu aufgebaut werden.

Nach vollständiger Applikation des Knochenzements kann die Applikationsvorrichtung 1 von der Kanüle 30 getrennt werden.

Nach Aushärten kann anschließend die Kanüle 30 leicht verkippt und gleichzeitig unter Zuhilfenahme der Angriffselemente 33 um ihre Längsachse 34 verdreht werden. Durch die schräge, geschliffene Kante 43 erfolgt dabei eine Abscherung des sich noch innerhalb des Rohrs 36 befindenden ausgehärteten Knochenzements, so daß bei einem anschließenden Herausziehen der Kanüle 30 dieses Material sicher zusammen mit der Kanüle 30 aus dem Körper entnommen wird.

### Bezugszeichenliste

- 1: Applikationsvorrichtung
- 2: Gehäuse
- 3: Zylinder
- 4: Inneres des Zylinders
- 5: Kolben
- 6: Längsachse
- 7: Abdichtungsmittel
- 8: Austrittsöffnung
- 9: Kopplungselement
- 10: Schaft
- 11: Schraubgewinde
- 12: Betätigungselement
- 13: Vertiefungen
- 14: Angriffsfläche
- 15: Handgriff
- 16: oberer Bereich des Handgriffs
- 17: Hohlraum
- 18: Verriegelungselement
- 19: Verschlußelement
- 20: Schraubenfeder
- 21: Entriegelungselement
- 22: Führungsausnehmung
- 23: Bolzen
- 24: Zahnstange
- 25: Zähne der Zahnstange (Gegenverzahnung)
- 26: Flanken
- 27: Flanken
- 28: Bohrung
- 29: Bohrung
- 30: Kanüle
- 31: proximales Endes der Kanüle
- 32: Kopplungselement
- 33: Angriffselemente
- 34: Längsachse der Kanüle
- 35: Mandrin
- 36: Rohr
- 37: proximales Ende des Mandrins
- 38: Abschlußelement
- 39: Schlagfläche
- 40: distales Ende des Mandrins
- 41: Öffnung
- 42: Spitze
- 43: Kante
- 44: Schrägfläche
- 45: Austrittsfläche
- 46: Stift
- 47: Ansatz
- 48: Hohlraum
- 49: Abstützfläche
- 50: stirnseitige Fläche des Kopplungselements
- 51: Schlitz
- 52: Längsabschnitt
- 53: Querabschnitt
- 54: freies Ende des Schlitzes 51
- 55: Rastausnehmung

## Patentansprüche

1. Kanüle für eine Applikationsvorrichtung (1) zum Applizieren von Knochenzement mit einer am distalen Ende (40) ausgebildeten Öffnung (41) und einem am proximalen Ende (31) vorgesehenen Kopplungsabschnitt (32) zum Ankoppeln an die Applikationsvorrichtung (1),
wobei das distale Ende (40) der Kanüle (30) bezüglich der Längsachse (34) der Kanüle (30) asymmetrisch ausgebildet ist, und
wobei am proximalen Ende (31) der Kanüle (30) seitlich nach außen ragende Angriffselemente (33) vorgesehen sind, mit denen die Kanüle (30) sowohl um ihre Längsachse (34) verdrehbar als auch entlang ihrer Längsrichtung verschiebbar ist,
**dadurch gekennzeichnet ,**
**daß** das distale Ende (40) der Kanüle (30) mit einer seitlich der Längsachse (34) liegenden Spitze (42) ausgebildet ist,
**daß** in die Kanüle (30) ein Mandrin (35) einsetzbar ist, und
**daß** in dem Bereich des proximalen Endes (31) der Kanüle (30) ein Verbindungselement (51) zur Erzeugung einer lösbaren und in axialer, distaler und proximaler Richtung verschiebefesten Verbindung zwischen dem Mandrin (35) und der Kanüle (30) vorgesehenen ist.

2. Kanüle nach Anspruch 1,
**dadurch gekennzeichnet,**
**daß** die die Umrandung der Öffnung (41) bildende Kante (43) des Kanülenendes (40) als Schneide geschliffen ist.

3. Kanüle nach Anspruch 1 oder 2,
**dadurch gekennzeichnet,**
**daß** sich die Durchtrittsfläche (45) der Öffnung (41) schräg zur Längsachse (34) der Kanüle (30) erstreckt.

4. Kanüle nach einem der vorhergehenden Ansprüche,
**dadurch gekennzeichnet ,**
**daß** zwei Angriffselemente (33) vorgesehen sind, die sich bezüglich der Längsachse (34) der Kanüle (30) insbesondere gegenüberliegend angeordnet sind.

5. Kanüle nach einem der vorhergehenden Ansprüche,
**dadurch gekennzeichnet,**
**daß** die Angriffselemente (33) als radial nach außen vorstehende stiftförmige Elemente ausgebildet sind.

6. Kanüle nach einem der vorhergehenden Ansprüche,
**dadurch gekennzeichnet ,**
**daß** das Verbindungselement (51) zur Erzeugung einer drehfesten Verbindung zwischen dem Mandrin (35) und der Kanüle (30) ausgebildet ist.

7. Kanüle nach einem der vorhergehenden Ansprüche,
**dadurch gekennzeichnet,**
**daß** die Ventile mit einem Mandrin (35) versehen ist, und **daß** an dem Mandrin (35), insbesondere im Bereich des proximalen Endes des Mandrins (35), ein mit dem Verbindungselement (51) zusammenwirkendes Gegenelement (46) vorgesehen ist.

8. Kanüle nach einem der vorhergehenden Ansprüche,
**dadurch gekennzeichnet ,**
**daß** die Verbindung zwischen dem Mandrin (35) und der Kanüle (30) durch einen Bajonettverschluß gebildet wird.

9. Kanüle nach einem der vorhergehenden Ansprüche,
**dadurch gekennzeichnet,**
**daß** das Verbindungselement als schlitzförmige Ausnehmung (51), insbesondere als Nut oder Durchbrechung, und das Gegenelement als insbesondere stiftförmiger Ansatz (46) oder umgekehrt ausgebildet sind.

10. Kanüle nach Anspruch 9,
**dadurch gekennzeichnet ,**
**daß** die Ausnehmung (51) zumindest einen in axialer Richtung der Kanüle (30) verlaufenden Längsabschnitt (52) umfaßt.

11. Kanüle nach Anspruch 10,
**dadurch gekennzeichnet ,**
**daß** sich an den Längsabschnitt (52) ein in Umfangsrichtung der Kanüle (30) verlaufender Querabschnitt (53) der Ausnehmung (51) anschließt.

12. Kanüle nach einem der vorhergehenden Ansprüche,
**dadurch gekennzeichnet,**
**daß** das Verbindungselement (51) an dem Kopplungsabschnitt (32) vorgesehen ist.

## Claims

1. A cannula for an application device (1) for the application of bone cement having an opening (41) formed at the distal end (40) and a coupling section (32) provided at the proximal end (31) for coupling to the application device (1),
wherein the distal end (40) of the cannula (30) is asymmetric with respect to the longitudinal axis (34) of the cannula (30); and
wherein laterally outwardly projecting engagement elements (33) are provided at the proximal end (31) of the cannula (30) with which the cannula (30) is both rotatable around its longitudinal axis (34) and displaceable along its longitudinal direction,
**characterised in that**
the distal end (40) of the cannula (30) is made with a tip (42) disposed to the side of the longitudinal axis (34);
**in that** a mandrel (35) can be inserted into the cannula (30); and
**in that** a connection element (51) is provided in the region of the proximal end (31) of the cannula (30) for the generation of a releasable connection, which is fixed with respect to displacement in the axial, distal and proximal directions, between the mandrel (35) and the cannula (30).

2. A cannula in accordance with claim 1, **characterised in that** the edge (43) of the cannula end (40) forming the peripheral rim of the opening (41) is ground as a cutting edge.

3. A cannula in accordance with claim 1 or claim 2, **characterised in that** the passage surface (45) of the opening (41) extends obliquely to the longitudinal axis (34) of the cannula (30).

4. A cannula in accordance with any one of the preceding claims, **characterised in that** two engagement surfaces (33) are provided which are in particular arranged oppositely disposed with respect to the longitudinal axis (34) of the cannula (30).

5. A cannula in accordance with any one of the preceding claims, **characterised in that** the engagement elements (33) are made as radially outwardly projecting, pin-like elements.

6. A cannula in accordance with any one of the preceding claims, **characterised in that** the connection element (51) is made for the generation of a rotationally fixed connection between the mandrel (35) and the cannula (30).

7. A cannula in accordance with any one of the preceding claims, **characterised in that** the cannula is provided with a mandrel (35); and
**in that** a counter element (46) cooperating with the connection element (51) is provided at the mandrel (35), in particular in the region of the proximal end of the mandrel (35).

8. A cannula in accordance with any one of the preceding claims, **characterised in that** the connection between the mandrel (35) and the cannula (30) is formed by a bayonet fastening.

9. A cannula in accordance with any one of the preceding claims, **characterised in that** the connection element is made as a slit-like cut-out (51), in particular as a groove or opening, and the counter-element is made as a nose (46), in particular a pin-shaped nose, or vice versa.

10. A cannula in accordance with claim 9, **characterised in that** the cut-out (51) includes at least one longitudinal section (52) extending in the axial direction of the cannula (30).

11. A cannula in accordance with claim 10, **characterised in that** a transverse section (53) of the cut-out (51) extending in the peripheral direction of the cannula (30) adjoins the longitudinal section (52).

12. A cannula in accordance with any one of the preceding claims, **characterised in that** the connection element (51) is provided at the coupling section (32).

## Revendications

1. Canule pour un dispositif d'application (1) destiné à appliquer du ciment osseux, comprenant une ouverture (41) réalisée à l'extrémité distale (40) et un tronçon d'accouplement (32) prévu à l'extrémité proximale (31) pour l'accouplement au dispositif d'application (1),
dans laquelle l'extrémité distale (40) de la canule (30) est réalisée asymétrique par rapport à l'axe longitudinal (34) de la canule (30), et
dans laquelle à l'extrémité proximale (31) de la canule (30) sont prévus des éléments d'engagement (33) qui font saillie latéralement vers l'extérieur et au moyen desquels la canule (30) est capable de tourner autour de son axe longitudinal (34) et de se déplacer en translation le long de sa direction longitudinale,
**caractérisée en ce que**
l'extrémité distale (40) de la canule (30) est réalisée avec une pointe (42) située latéralement par rapport à l'axe longitudinal (34),
**en ce qu'**un mandrin (35) est susceptible d'être mis en place dans la canule (30), et
un élément de liaison (51), destiné à produire une liaison détachable mais sans déplacement en direction axiale, en direction distale et en direction proximale entre le mandrin (35) et la canule (30), est prévu dans la région de l'extrémité proximale (31) de la canule (30).

2. Canule selon la revendication 1,
**caractérisée en ce que** l'arête (43) de l'extrémité de canule (40), qui forme la bordure de l'ouverture (41), est meulée comme un tranchant.

3. Canule selon la revendication 1 ou 2,
**caractérisée en ce que** la surface de passage (45) de l'ouverture (41) s'étend en oblique par rapport à l'axe longitudinal (34) de la canule (30).

4. Canule selon l'une des revendications précédentes,
**caractérisée en ce qu'**il est prévu deux éléments d'engagement (33) qui sont agencés en particulier en opposition par rapport à l'axe longitudinal (34) de la canule (30).

5. Canule selon l'une des revendications précédentes,
**caractérisée en ce que** les éléments d'engagement (33) sont réalisés comme des éléments en forme de tiges qui dépassent radialement vers l'extérieur.

6. Canule selon l'une des revendications précédentes,
**caractérisée en ce que** l'élément de liaison (51) est réalisé en vue d'engendrer une liaison solidaire en rotation entre le mandrin (35) et la canule (30).

7. Canule selon l'une des revendications précédentes,
**caractérisée en ce que** la canule est pourvue d'un mandrin (35), et **en ce qu'**un élément antagoniste (46), qui coopère avec l'élément de liaison (51), est prévu sur le mandrin (35), en particulier dans la zone de l'extrémité proximale du mandrin (35).

8. Canule selon l'une des revendications précédentes,
**caractérisée en ce que** la liaison entre le mandrin (35) et la canule (30) est formée par une fermeture à baïonnette.

9. Canule selon l'une des revendications précédentes,
**caractérisée en ce que** l'élément de liaison est réalisé sous la forme d'un évidement (51) en forme de fente, en particulier comme une gorge ou une traversée, et l'élément antagoniste est réalisé sous la forme d'un talon (46), en particulier en forme de tige, ou inversement.

10. Canule selon la revendication 9,
**caractérisée en ce que** l'évidement (51) comprend au moins un tronçon longitudinal (52) qui s'étend en direction axiale de la canule (30).

11. Canule selon la revendication 10,
**caractérisée en ce qu'**un tronçon transversal (53) de l'évidement (51), qui s'étend en direction périphérique de la canule (30), se raccorde au tronçon allongé (52).

12. Canule selon l'une des revendications précédentes,
**caractérisée en ce que** l'élément de liaison (51) est prévu sur le tronçon d'accouplement (32).
